# EUROPEAN PATENT SPECIFICATION

(11) **EP 1 696 845 B1**
(45) Date of publication and mention of the grant of the patent: **18.02.2009**
(21) Application number: 04815239.1
(22) Date of filing: 23.12.2004
(51) Int. Cl.: A61F 9/01

(54) **HYBRID EYE TRACKING SYSTEM AND ASSOCIATED METHODS**
HYBRID-AUGENVERFOLGUNGSSYSTEM UND VERWANDTE VERFAHREN
SYSTEME DE SUIVEUR OCULAIRE HYBRIDE ET PROCEDES ASSOCIES

(30) Priority: 23.12.2003 US 532041 P
(43) Date of publication of application: 06.09.2006
(73) Proprietor: Alcon RefractiveHorizons, Inc., Fort Worth, Texas 76134 (US)
(72) Inventor: GRAY, Gary, Orlando, FL 32837 (US); CAMPIN, John, A., Orlando, FL 32828 (US); BOTT, Steven, E., Oviedo, FL 32765 (US); GIBBS, David, Michael, Orlando, FL 32832 (US); PETTIT, George, H., Maitland, FL 32751 (US)
(74) Representative: Hanna, Peter William Derek
(86) International application number: PCT/US2004/043130
(87) International publication number: WO 2005/063154

(56) References cited:
- US-A- 5 098 426
- US-A1- 2002 013 577
- US-B1- 6 280 436
- US-B1- 6 607 527
- US-B1- 6 658 282
- ANONYMOUS: "What is true custom ablation?" EUROTIMES, [Online] vol. supplement, October 2002 (2002-10), pages 1-20, XP002339208 Dublin Retrieved from the Internet: URL:http://www.escrs.org/eurotimes/october 2002/supplement/supplement.asp> [retrieved on 2005-08-02]

## Description

### FIELD OF THE INVENTION

The present invention relates to systems and methods for performing eye tracking, such as during laser surgery, and, more particularly, to such systems for tracking both rapid and slow eye movements.

### DESCRIPTION OF RELATED ART

The measurement of wavefront aberrations emanating from an eye is known in the art to provide data for driving laser-surgical corrective systems. Various embodiments of a method and system for objectively measuring and surgically correcting aberrations of optical systems by wavefront analysis have been disclosed in commonly owned application WO 01/85016, "Apparatus and Method for Objective Measurement and Correction of Optical Systems Using Wavefront Analysis, "filed May 8,2000, and in commonly owned issued U. S. Patent Nos. 5,632, 742 and 5,980, 513.

Corrective laser surgery may be performed, for example, by laser ablation of portions of the corneal surface to achieve a calculated shape for improving visual acuity. In this case it is desirable to account for eye movement during surgery while delivering laser shots to the cornea. Rapid, involuntary ("saccadic") eye movement comprises motion of very short duration, in the 10-20 msec range, and up to 10 of rotation. This movement makes it difficult to determine a visual axis from which to calculate a movement of a treatment laser.The'513 patent addresses this problem with an x,y eye tracking system such as will be described more fully in the following.

Two other types of eye movement, which are slower than saccadic eye movement, comprise cyclo-torsion and translation movements. Translation movement is at present eliminated by paralyzing the pupil so that asymmetric pupil dilation and constriction do not shift the tracking point, resulting in decentration.

US6607527 relates to a system for precision laser surgery in which monitors movement of the eye and conveys eyetracking information to the laser delivery system including a non-invasive eye tilt reference marker; "What is true custom ablation?" Eurotimes, vol. supplement, October 2002 (2002-10) Dublin, describes a closed loop eye tracking arrangement; and US5098426 describes an apparatus for laser precision surgery comprising a tracking system for tracking movement of the eye during surgery. The tacking means includes a servo mirror which allows closed loop tracking via the microprocessor.

### SUMMARY OF THE INVENTION

It is therefore an object of the present invention to provide a system for tracking eye movement in particular a system usable during laser surgery to correct ocular aberrations.

It is a further object to provide a system for tracking eye movement that obviates the need for eye dilation during laser surgery.

It is another object to provide a system for tracking eye movement that obviates the need for paralyzing the patient's pupil during laser surgery.

It is also an object to provide a system for tracking both rapid and slow eye movement.

It is an additional object to provide a system for automating adjustments to laser beam delivery parameters.

These and other objects are achieved by the present invention, a hybrid eye tracking system. The system can be used for tracking ocular changes an eye-safe optical beam being directed toward an eye. The eye can be substantially untreated (minimally treated by drugs or otherwise) to achieve dilation and paralysis. Next a reflected optical beam from the eye is detected, and a plurality of measurements are performed based upon data contained in the reflected optical beam. The measurements are made of at least one geometric parameter of the eye at a predetermined frequency, and from them is calculated a change in the at least one geometric parameter.

Systems are provided according to the present invention for performing the above-described methods.

The features that characterize the invention, together with further objects and advantages thereof, will be better understood from the following description used in conjunction with the accompanying drawing. It is to be expressly understood that the drawing is for the purpose of illustration and description and is not intended as a definition of the limits of the invention. These and other objects attained, and advantages offered, by the present invention will become more fully apparent as the description that now follows is read in conjunction with the accompanying drawing.

### BRIEF DESCRIPTION OF THE DRAWINGS

A more complete understanding of the present invention and the advantages thereof may be acquired by referring to the following description, taken in conjunction with the accompanying drawings in which like reference numbers indicate like features and wherein:
FIGURE 1 is a schematic diagram of the hybrid eye tracking system of the present invention;
FIGURE 2 is a flow diagram of the slow movement tracker control system of the present invention; and
FIGURE 3 is a schematic diagram of an eye, illustrating the parameters monitored by the system.

### DETAILED DESCRIPTION OF THE PREFERRED EMBODIMENTS

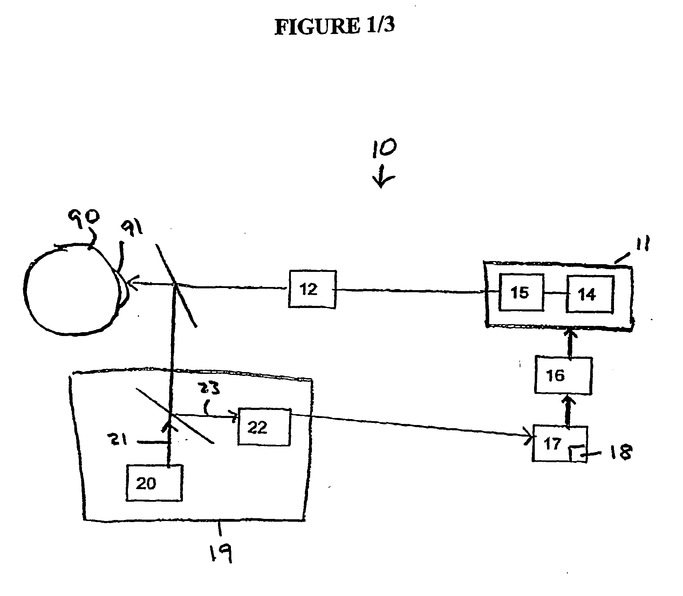

A description of the preferred embodiments of the present invention will now be presented with reference to FIGS. 1-3.

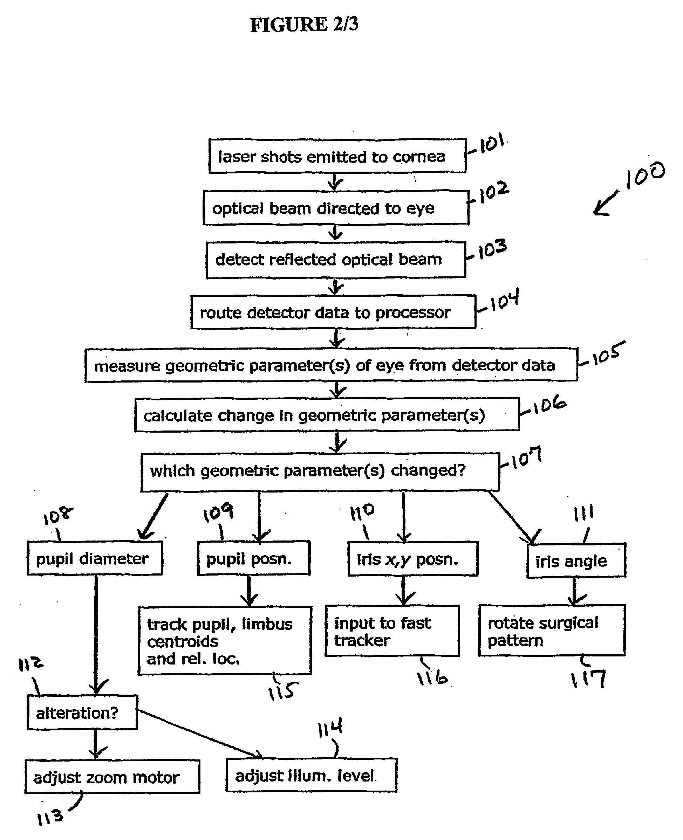

A system 10 (FIG. 1) and method 100 (FIG. 2) for performing a corrective procedure upon an undilated, unparalyzed eye are provided. The system 10 comprises a surgical component 11 and a tracking component, which in turn comprises a rapid eye movement (saccadic) tracker 12, such as disclosed in the '513 patent, and a slow movement tracker 13. The tracking components 12,13 are for tracking ocular changes during the surgical procedure.

The surgical component 11 comprises an ablating laser 14 and associated optics 15 adapted for emitting laser beam shots at a cornea 91 of an eye 90 in a predetermined pattern (block 101) based upon, for example, a wavefront measurement, although this is not intended as a limitation. The surgical component 11 is under control of a beam translation component 16 that is in turn under control of a processor 17 having software 18 resident thereon. The software 18 comprises means for performing calculations on data received from the tracking components 12,13.

The slow tracker 13 comprises a video system 19 comprising an illumination means 20 adapted to direct an eye-safe optical beam 21 toward the eye 90 (block 102). The video system 19 further comprises a detector 22 for detecting a reflected optical beam 23 from the eye 90 (block 103), tracking features such as, for example, scleral features such as blood vessels, limbus shape and ellipticity, iris features, and artificial eye marks. Data from the detector 22 are routed to the processor 17 (block 104), from which a plurality of measurements are made of at least one geometric parameter of the eye 90 at a predetermined frequency (block 105), in one embodiment preferably less than 250 Hz. From these measurements is calculated a change in the at least one geometric parameter (block 106), including a translational and/or a cyclo-torsional change.

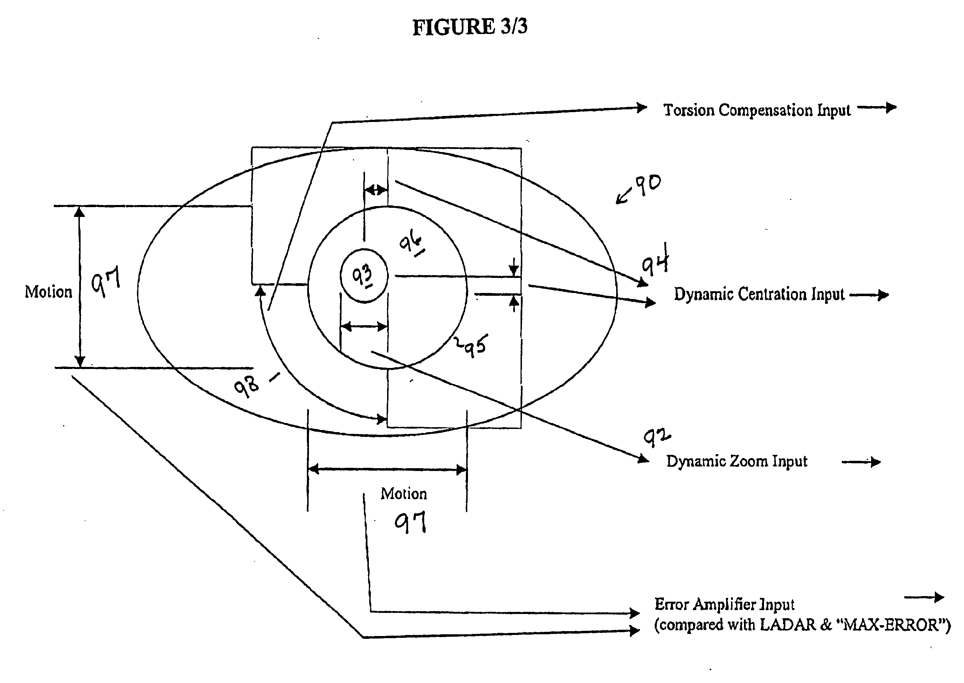

The geometric parameter (see FIG. 3) may comprise (block 107), for example, at least one of a diameter 92 of a pupil 93 (block 108), a pupil (centroid) position 94 relative to the limbus 95 (block 109), and a position of the iris 96 in *x*, *y* coordinates 97 (block 110) and as a cyclo-torsion angle 98 (block 111).

If the pupil diameter 92 changes, either (block 112) the zoom motor 24 adjusts (block 113) or the ambient illumination level in the room is adjusted to maintain a substantially constant pupil diameter 92 (block 114). Preferably this measurement is updated at a rate approximately 10 times the rate of change of the pupil diameter 92.

If the pupil position 94 changes relative to the limbus 95, an *x,y* offset is calculated from the limbus 95, and the centroids of the pupil 93 and the limbus 95 are tracked, along with their relative locations (block 115). Preferably this measurement is updated at a rate approximately 10 times the rate of change of the pupil diameter 92. Pupil centroid offset or "pupil drift" depends on the state of the iris and may be larger or smaller from patient to patient and may increase or decrease slightly during surgery. This displacement may not be noticed by the fast iris/pupil boundary tracker and may need to be added vectorally to every shot position with the excimer scanner mirrors so that the shots end up on the desired locations on the cornea independent of pupil centroid drift.

A preferred way to account for pupil center drift due to pupil size changes is to characterize the pupil center position relative to the center of the limbus at the pre-operative visit using the video images of the pupil under different lighting conditions. An equation for how pupil center shifts with respect to size changes of the pupil can be developed from such a pre-operative evaluation. It is assumed that during surgery, if the video tracker observed pupil size changes, the same drift offset will be present. This means that an equal and opposite offset in the location of where the excimer scanners will place these shots must be applied to "null out" the effect that the pupil drift would have on the high speed tracked position, which is only closed around the real-time pupil center and will not account for changes in relative position of the pupil center with respect to the limbus or cornea.

If the iris position in *x*, *y* coordinates 97 changes relative to the excimer laser center, due to, for example, the normal head shifts, chin rolling or other movements that cause the entire eye to move, the eye will be tracked-in using the high-speed four IR spot closed-loop LADAR tracker, which uses the tracking galvonometer mirrors to constantly adjust the position of the tracked eye to maintain a space stabilized image

If the rotational position 98 of the iris 96 changes, the new position is used to calculate and perform a rotation of the surgical pattern generated by the excimer scanning mirrors to compensate for cyclo-torsion (block 117). Video tracking may be performed using iris feature recognition, scleral blood vessel detection 96 or on features marked on the eye 90. Preferably this measurement is updated at a rate 10 times the rate of change of the ocular rotation.

Ocular changes noted on the video images that cause the locations on the cornea of the desired laser ablation pattern to be shifted relative the location of the excimer beam scanning positions are compensated for by dynamically adjusting the directing of the laser beam shots via the excimer laser scanning mirrors based upon the calculated change.

It will be seen by one of skill in the art that other embodiments and uses may be contemplated for the present invention. For example, also the eye tracker monitoring system may be used in settings other than surgical sites, including such sites as for psychological and physiological testing application,

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the apparatus illustrated and described herein are by way of example, and the scope of the invention is not limited to the exact details of construction.

Having now described the invention, the construction, the operation and use of preferred embodiment thereof, and the advantageous new and useful results obtained thereby, the new and useful constructions, and reasonable mechanical equivalents thereof obvious to those skilled in the art, are set forth in the appended claims.

## Claims

1. A system for performing a corrective procedure upon an eye comprising a laser ablating means (14) and beam control means (16) for directing a plurality of ablating laser beam shots at a cornea (91) of an undilated, unparalyzed eye (90) in a predetermined pattern, and tracking means (10) for tracking ocular changes (12, 13) during a surgical procedure, wherein the tracking means comprises
a slow movement tracking means (13) comprising means for directing an eye-safe optical beam (21) toward an undilated, unparalyzed eye (90); a video detector (22) for collecting reflected optical beam data from the eye at a predetermined frequency (105); and
software means (18) installable on a processor (17) for calculating from the collected optical beam data a change in at least one geometric parameter (106) of the eye;
a saccadic eye movement tracking means (12) for tracking rapid eye movement using the boundary between the pupil and an iris of the eye; wherein the software means (18) uses the calculated change in the at least one geometric parameter (106) of the eye as input for adjusting the saccadic eye movement tracker; and
the software means (18) being operable to perform calculations on the data received from the slow and saccadic eye movement tracking means (12,13) to compensate for ocular changes by dynamically adjusting the directing of the laser beam shots based upon the calculated changes.

2. The system recited in Claim 1, wherein the eye safe optical beam (21) comprises a Class 1 Laser.

3. The system recited in Claim 1, wherein the detector (22) comprises a video monitor (19).

4. The system recited in Claim 3, wherein the video monitor (19) is adapted to collect data at a detection rate less than 250 Hz.

5. The system recited in Claim 1, wherein the software means (18) is adapted to determine at least one of a cyclo-torsion and a translation of an eye component.

6. The system recited in Claim 1, wherein the saccadic eye movement tracker (12) is a laser-based eye movement tracker.

7. The system recited in Claim 1, wherein the at least one geometric parameter comprises a rotation (111) of an iris of the eye.

8. The system recited in Claim 7, wherein the adjusting means comprises means for rotating the predetermined pattern (117) to compensate for the iris rotation.

9. The system recited in Claim 1, wherein the at least one geometric parameter comprises a position of a pupil (109) of the eye relative to an iris of the eye.

10. The system recited in Claim 9, wherein the adjusting means comprises means for translating (115) the predetermined pattern to compensate for the pupillary position.

11. The system recited in Claim 1, wherein the at least one geometric parameter comprises a diameter of a pupil (108) of the eye.

12. The system recited in Claim 11, wherein the adjusting means comprises means for providing input (116) for the saccadic eye movement tracking.

13. The system recited in Claim 1, further comprising means for adjusting a level of illumination (114) surrounding the eye to maintain a substantially constant pupil diameter.

14. The system recited in Claim 1, wherein the at least one geometric parameter comprises a position of a limbus of the eye.

15. The system recited in Claim 14, wherein the adjusting means comprises means for providing input (116) for the saccadic eye movement tracking.

16. A method (100) oftracking ocular changes in settings other than surgical sites, the method comprising the steps of:
directing (102) an eye-safe optical beam (21) toward an undilated, unparalyzed eye (90);
detecting (103) a reflected optical beam from the eye;
routing detected data to a processor (17) comprising software means (18)
the processor (17) performing a plurality of measurements (105) based upon data contained in the reflected optical beam of at least one geometric parameter of the eye at a predetermined frequency;
the software means (18) calculating (106) from the measurements a change in the at least one geometric parameter;
tracking saccadic eye movements; and using (116) the calculated change in the at least one geometric parameter as input for adjusting the saccadic eye movement tracking,

17. The method recited in Claim 16, wherein the geometric parameter comprises at least one of a pupil diameter (108), a pupil position (109) relative to a limbus of the eye, and an iris position (110).

18. The method recited in Claim 16, wherein the optical beam (21) comprises a Class 1 Laser.

19. The method recited in Claim 16, wherein the detecting step (103) comprises performing video monitoring.

20. The method recited in Claim 19, wherein the video monitoring step is performed at a detection rate less than 250 Hz.

21. The method recited in Claim 16, wherein the calculating step (106) comprises determining at least one of a cyclo-torsion and a translation of an eye component.

## Patentansprüche

1. System zur Durchführung eines Nachstellablaufes auf ein Auge, welches ein Laserablationsmittel (14) und ein Strahlsteuermittel (16) zum Richten von einer Mehrzahl von Ablationslaserstrahlstößen auf eine Hornhaut (91) von einem ungedehnten, ungelähmten Auge (90) in ein vorbestimmtes Muster, und ein Nachführmittel (10) zum Nachführen von okularen Änderungen (12, 13) während einer Operation enthält, wobei das Nachführmittel enthält:
ein Nachführmittel (13) einer langsamen Bewegung, welches ein Mittel zum Richten eines augensicheren Optikstrahls (21) auf ein ungedehntes, ungelähmtes Auge (90) enthält; einen Videodetektor (22) zum Einsammeln von reflektierten Optikstrahldaten von dem Auge bei einer vorbestimmten Frequenz (105); und
ein Softwaremittel (18), welches auf einem Prozessor (17) installierbar ist, um anhand der eingesammelten Optikstrahldaten eine Änderung in zumindest einem geometrischen Parameter (106) von dem Auge zu berechnen;
ein Bewegungsnachführmittel (12) eines sakkadischen Auges zum Nachführen einer schnellen Augenbewegung unter Verwendung der Grenze zwischen der Pupille und einer Iris von dem Auge; wobei das Softwaremittel (18) die berechnete Änderung in dem zumindest einen geometrischen Parameter (106) von dem Auge als eine Eingabe zum Einstellen des Bewegungsnachführers eines sakkadischen Auges verwendet; und
wobei das Softwaremittel (18) dazu betriebsbereit ist, um Berechnungen auf die Daten durchzuführen, welche von den Nachführmitteln (12, 13) einer langsamen Augenbewegung und einer sakkadischen Augenbewegung empfangen sind, um okulare Änderungen durch ein dynamisches Einstellen der Richtung der Laserstrahlstöße basierend auf den berechneten Änderungen zu kompensieren.

2. System nach Anspruch 1, bei welchem der augensichere Optikstrahl (21) einen Klasse-1-Laser enthält.

3. System nach Anspruch 1, bei welchem der Detektor (22) einen Videomonitor (19) enthält.

4. System nach Anspruch 3, bei welchem der Videomonitor (19) dazu ausgelegt ist, um Daten bei einer Erfassungsrate von weniger als 250 Hz einzusammeln.

5. System nach Anspruch 1, bei welchem das Softwaremittel (18) dazu ausgelegt ist, um zumindest eines aus einer Wechseltorsion und einer Translation von einem Augenbestandteil zu bestimmen.

6. System nach Anspruch 1, bei welchem der Bewegungsnachführer (12) eines sakkadischen Auges ein laserbasierter Augenbewegungs-Nachführer ist.

7. System nach Anspruch 1, bei welchem der zumindest eine geometrische Parameter eine Umdrehung (111) von einer Iris von dem Auge enthält.

8. System nach Anspruch 7, bei welchem das Einstellmittel ein Mittel zum Umdrehen des vorbestimmten Musters (117) enthält, um die Irisumdrehung zu kompensieren.

9. System nach Anspruch 1, bei welchem der zumindest eine geometrische Parameter eine Position von einer Pupille (109) von dem Auge in Relation zu einer Iris von dem Auge enthält.

10. System nach Anspruch 9, bei welchem das Einstellmittel ein Mittel zum Überführen (115) des vorbestimmten Musters enthält, um die Pupillarposition zu kompensieren.

11. System nach Anspruch 1, bei welchem der zumindest eine geometrische Parameter einen Durchmesser von einer Pupille (108) von dem Auge enthält.

12. System nach Anspruch 11, bei welchem das Einstellmittel ein Mittel zum Bereitstellen von einer Eingabe (116) für die Bewegungsnachführung des sakkadischen Auges enthält.

13. System nach Anspruch 1, welches ferner ein Mittel zum Einstellen von einem Pegel einer Beleuchtung (114), welche das Auge umgibt, enthält, um einen im Wesentlichen konstanten Pupillendurchmesser beizubehalten.

14. System nach Anspruch 1, bei welchem der zumindest eine geometrische Parameter eine Position von einem Limbus von dem Auge enthält.

15. System nach Anspruch 14, bei welchem das Einstellmittel ein Mittel zum Bereitstellen von einer Eingabe (116) für die Bewegungsnachführung des sakkadischen Auges enthält.

16. Verfahren (100) zum Nachführen von okularen Änderungen bei Einstellungen, welche sich von Operationsstellen unterscheiden, wobei das Verfahren die Schritte enthält:
Richten (102) eines augensicheren Optikstrahls (21) auf ein ungedehntes, ungelähmtes Auge (90);
Erfassen (103) eines reflektierten Optikstrahls von dem Auge;
Weiterleiten von erfassten Daten an einen Prozessor (17), welcher ein Softwaremittel (18) enthält, wobei der Prozessor (17) eine Mehrzahl von Messungen (105) basierend auf Daten, welche in dem reflektierten Optikstrahl von zumindest einem geometrischen Parameter von dem Auge enthalten sind, bei einer vorbestimmten Frequenz durchführt;
wobei das Softwaremittel (18) anhand der Messungen eine Änderung in dem zumindest einen geometrischen Parameter berechnet (106);
Nachführen von sakkadischen Augenbewegungen; und Verwenden (116) der berechneten Änderung in dem zumindest einen geometrischen Parameter als eine Eingabe zum Einstellen der Bewegungsnachführung des sakkadischen Auges.

17. Verfahren nach Anspruch 16, bei welchem der geometrische Parameter zumindest eines enthält aus einem Pupillen-Durchmesser (108), eine Pupillen-Position (109) in Relation zu einem Limbus von dem Auge, und eine Iris-Position (110).

18. Verfahren nach Anspruch 16, bei welchem der Optikstrahl (21) einen Klasse-1-Laser enthält.

19. Verfahren nach Anspruch 16, bei welchem der Erfassungsschritt (103) eine Durchführung von einer Videoüberwachung enthält.

20. Verfahren nach Anspruch 19, bei welchem der Videoüberwachungsschritt bei einer Erfassungsrate von weniger als 250 Hz durchgeführt wird.

21. Verfahren nach Anspruch 16, bei welchem der Berechnungsschritt (106) ein Bestimmen von zumindest einem aus einer Wechseltorsion und einer Translation von einem Augen-Bestandteil enthält.

## Revendications

1. Système pour effectuer un processus de correction sur un oeil, comprenant des moyens d'ablation à laser (14) et des moyens de contrôle de faisceau (16) pour diriger une pluralité de décharges de faisceau laser d'ablation au niveau d'une cornée (91) d'un oeil non paralysé et non dilaté (90) selon un motif prédéterminé, et des moyens de suivi (10) pour suivre des changements oculaires (12, 13) pendant un processus chirurgical, les moyens de suivi comportant :
des moyens de suivi de mouvement lent (13) comprenant des moyens pour diriger un faisceau optique sûr pour l'oeil (21) vers un oeil non dilaté et non paralysé (90), un détecteur vidéo (22) pour collecter des données de faisceau optique réfléchi depuis l'oeil à une fréquence prédéterminée (105), et
des moyens formant logiciel (18) pouvant être installés sur un processeur (17) pour calculer, à partir des données de faisceau optique recueillies, un changement d'au moins un paramètre géométrique (106) de l'oeil,
des moyens de suivi de mouvement d'oeil saccadé (12) pour suivre un mouvement d'oeil rapide en utilisant la limite entre la pupille et l'iris de l'oeil, les moyens formant logiciel (18) utilisant le changement calculé du au moins un paramètre géométrique (106) de l'oeil en tant qu'entrée pour ajuster le dispositif de suivi de mouvement d'oeil saccadé, et
les moyens formant logiciel (18) étant opérationnels pour réaliser des calculs sur les données reçues à partir des moyens de suivi de mouvement d'oeil lents et saccadé (12, 13) pour compenser des changements oculaires en ajustant de manière dynamique la direction des décharges de faisceau laser sur la base des changements calculés.

2. Système selon la revendication 1, dans lequel le faisceau optique sûr pour l'oeil (21) comprend un laser de catégorie 1.

3. Système selon la revendication 1, dans lequel le détecteur (22) comprend un moniteur vidéo (19).

4. Système selon la revendication 3, dans lequel le moniteur vidéo (19) est adapté pour collecter des données à une vitesse de détection inférieure à 250 Hz.

5. Système selon la revendication 1, dans lequel les moyens formant logiciel (18) sont adaptés pour déterminer au moins un élément parmi une cyclotorsion et une translation d'un composant oculaire.

6. Système selon la revendication 1, dans lequel le dispositif de suivi de mouvement d'oeil saccadé (12) est un dispositif de suivi de mouvement d'oeil à base de laser.

7. Système selon la revendication 1, dans lequel le au moins un paramètre géométrique comprend une rotation (11) de l'iris de l'oeil.

8. Système selon la revendication 7, dans lequel les moyens d'ajustement comprennent des moyens pour faire tourner le motif prédéterminé (117) afin de compenser une rotation de l'iris.

9. Système selon la revendication 1, dans lequel au moins un paramètre géométrique comprend une position de la pupille (109) de l'oeil par rapport à l'iris de l'oeil.

10. Système selon la revendication 9, dans lequel les moyens d'ajustement comprennent des moyens pour translater (104) le motif prédéterminé pour une compensation de la position de pupille.

11. Système selon la revendication 1, dans lequel le au moins un paramètre géométrique comprend un diamètre de la pupille (108) de l'oeil.

12. Système selon la revendication 11, dans lequel les moyens d'ajustement comprennent des moyens pour fournir une entrée (116) pour un suivi de mouvement d'oeil saccadé.

13. Système selon la revendication 1, comportant en outre des moyens pour ajuster un niveau d'éclairage (114) entourant l'oeil pour maintenir un diamètre de pupille sensiblement constant.

14. Système selon la revendication 1, dans lequel le au moins un paramètre géométrique comprend une position d'un limbe de l'oeil.

15. Système selon la revendication 14, dans lequel les moyens d'ajustement comprennent des moyens pour fournir une entrée (116) pour le suivi de mouvement d'oeil saccadé.

16. Procédé (100) de suivi de changements oculaires dans des installations autres que des sites chirurgicaux, le procédé comportant les étapes consistant à :
diriger (102) un faisceau optique sûr pour l'oeil (21) vers un oeil non paralysé et non dilaté (90),
détecter (103) un faisceau optique réfléchi par l'oeil,
router des données détectées vers un processeur (17) comprenant des moyens formant logiciel (18),
le processeur (17) effectuant une pluralité de mesures (105) sur la base de données contenues dans le faisceau optique réfléchi du au moins un paramètre géométrique de l'oeil à une fréquence prédéterminée,
les moyens formant logiciel (18) calculant (106) un changement du au moins un paramètre géométrique à partir des mesures,
suivre des mouvements d'oeil saccadés, et utiliser (116) le changement calculé du au moins un paramètre géométrique en tant qu'entrée pour ajuster le suivi de mouvement d'oeil saccadé.

17. Procédé selon la revendication 16, dans lequel le paramètre géométrique comprend au moins un élément parmi un diamètre de pupille (108), une position de pupille (109) par rapport à un limbe de l'oeil, et une position d'iris (10).

18. Procédé selon la revendication 16, dans lequel le faisceau optique (21) comprend un laser de catégorie 1.

19. Procédé selon la revendication 16, dans lequel l'étape de détection (103) comprend la mise en oeuvre d'une surveillance vidéo.

20. Procédé selon la revendication 19, dans lequel l'étape de surveillance vidéo est effectuée à une vitesse de détection inférieure à 250 Hz.

21. Procédé selon la revendication 16, dans lequel l'étape de calcul (106) comprend la détermination d'au moins un élément parmi une cyclo-torsion et une translation d'un composant oculaire.
